# EUROPEAN PATENT SPECIFICATION

(11) **EP 2 704 782 B1**
(45) Date of publication and mention of the grant of the patent: **18.01.2017**
(21) Application number: 12723845.9
(22) Date of filing: 04.05.2012
(51) Int. Cl.: A61M 16/04, A61M 29/02, A61B 17/00

(54) **DILATATION SYSTEM FOR A MEDICAL DEVICE**
DILATATIONSSYSTEM FÜR EINE MEDIZINISCHE VORRICHTUNG
SYSTÈME DE DILATATION POUR DISPOSITIF MÉDICAL

(30) Priority: 06.05.2011 IT GO20110004
(43) Date of publication of application: 12.03.2014
(73) Proprietor: Guerra, Romano, 34074 Monfalcone (GO) (IT)
(72) Inventor: GUERRA, Romano, I-34074 Monfalcone (GO) (IT); ALBALAT, Alberto Martinez, E-28864 Ajalvir Madrid (ES)
(74) Representative: Jacob, Reuben Ellis
(86) International application number: PCT/EP2012/058308
(87) International publication number: WO 2012/152725

(56) References cited:
- WO-A1-2005/072804
- WO-A1-2008/034872
- US-A- 5 058 580
- US-A1- 2004 255 954
- US-A1- 2010 160 947
- US-B2- 6 953 431

## Description

The present invention relates to a dilatation system for a medical device, in particular where the insertion of a tubular structure (e.g. cannula, catheter or drainage tube) is required. The dilatation system is ideally used for tracheostomy procedures, such as Percutaneous Dilating Tracheostomy (PDT). This minimally invasive surgical device enables to open a gap between the first tracheal rings and to introduce a tracheostomy tube in the trachea of a patient.

### BACKGROUND

Classical surgical tracheostomy is still the oldest and most frequently used procedure. The procedure consists of making a surgical gap between the rings of the trachea by using traumatic surgical instruments like scalpels, lancets and dilating pincers. All of this requires the involvement of a team of surgeons, a sterilized environment and complicated arrangements which are not always easy to obtain in emergency situations. To circumvent these disadvantages, in recent years new techniques and micro-invasive surgical instruments have been developed for Percutaneous Dilating Tracheostomy (PDT).

One of these techniques requires the use of dilating instruments made of water-repellent plastic material. These dilators of increasing dimensions are introduced between the first tracheal rings through a small opening made with a thick needle or with a scalpel. A guide wire is then inserted to provide guidance for the insertion of the dilator and to obtain a breach of size sufficient tube to allow placement of the tracheal tube. This technique requires a significant force to be applied along the longitudinal axis and towards the trachea's posterior wall. The disadvantages of this technique are the long sequence of manipulations, a significant manual effort, and the potential damage to the posterior wall of the trachea from friction with dilators during the insertion phase.

A modified form of this technique uses a curved cone-shaped dilator, similar to a rhinoceros horn, and has proved itself to be an advance on the previous technique by reducing the time required for the procedure but not by decreasing the risks of complications, including rupture of the tracheal rings.

Another method uses a plastic instrument shaped like a threaded cone. This device, like those described above, is inserted along a guide wire and, rotated in clockwise fashion, allows a gap to be opened between the tracheal rings. This method is more traumatic than the previous ones, with a higher incidence of complications, including frequent rupture of the tracheal rings. These techniques require the insertion of an instrument to position the tracheostomy tube after dilating and opening of a passage between the tracheal rings.

Two further systems have recently been proposed for PDT using a dilating balloon made of polyamide materials (PET or nylon) able to withstand the high pressures needed to open a gap during inflation between the tracheal rings.

The device described in US2004/0255954 uses a cylindrical balloon positioned in front, some 10 - 15 mm from the tracheostomy tube, and supported by a catheter aligned with the tube. In this case the balloon acts only as a dilator and the tube is positioned after the balloon has been deflated, with the tracheostomy tube being introduced by means of the insertion device fitted with a cone which exits from the opening of the tube. The limits of this system are most evident between the dilating and insertion stages, when a gaping opening is created, entailing possible bleeding from tissues or difficulty with introducing the tube due to tissue recoil.

A second device described in WO 2008/034872 uses a dilating balloon having a special reverse truncated cone shape, i.e. with the distal end of the balloon being larger than its proximal end. The balloon is partly contained within the tube which becomes an integral part of the system, being firmly anchored between the balloon and the handle. In this case, the balloon acts as both a dilator and introducer, since the balloon is introduced together with the tube immediately after dilatation. The balloon's special shape and the fact that its inflation begins at the distal wall facilitates introduction of the tracheostomy tube into the trachea.

US 6,953,431 describes a dilation balloon used in endoscopy to dilate strictures. The proximal end of the balloon has a concave shape in order to increase the contact area between the endoscope face and the balloon and assists with alignment engagement with the distal end of the endoscope. The balloon also comprises a flap like structure to provide protection against tissue or materials migrating into the space between the endoscope and the balloon.

It has however been observed that during the inflation stage, skin and paratracheal tissues had a tendency to become trapped between the proximal end of the partly inflated balloon and the distal end of the tracheal tube, thereby preventing further forward movement of the tube into the trachea. The risk is therefore that force must be applied to force the tube forward, thereby causing trauma and tissue damage to the insertion point.

### BRIEF DESCRIPTION OF THE INVENTION

The scope of the invention is as defined by the appended claims.

According to a first aspect of the invention, there is provided a dilatation system for a medical device comprising a tubular portion and a dilating balloon at the distal end of the tubular portion. The dilatation system according to the present invention comprises an insertion element for preventing skin or tissue to be trapped between the distal end of the tubular portion and the proximal end of the inflated balloon.

The insertion means comprises an insertion element to facilitate the transition from the balloon to the tubular structure to be inserted preventing skin or tissue to be trapped between the distal end of the intermediate tubular portion and the proximal end of balloon when this is inflated.

The inventor has developed a new tracheostomy device and a dilatation system that are very effective for dilating the tracheal rings and are improvements on previous systems. This new system facilitates introduction of the tracheostomy tube, avoiding problems with the patient's skin and the balloon while it is being inflated.

In its deflated state, the profile of proximal end of the balloon is generally smaller than the diameter of the distal end of the intermediary tubular portion. During the tracheostomy procedure, the patient's skin is punctured and the opening is dilated to enable passage, firstly of the deflated balloon and then once inflated the part of the intermediary tubular portion carrying the tracheostomy tube. The stretched skin and tissue have a tendency to contract themselves back into their original position after passage of the deflated balloon and to become trapped between the proximal end of the inflated balloon and the distal end of the intermediary tubular portion. Consequently, the tracheostomy device is prevented from being inserted further, unless considerable force is applied to push past the distal end of the intermediate tubular portion. The present invention prevents this blockage and allows a smooth and gradual insertion of the tracheal tube.

In a first embodiment, the insertion means comprises an insertion element located at the distal end of the tubular portion to be inserted. Preferably, the insertion element is located co-axially relative to the tubular portion inside the dilating balloon. The insertion element has a diameter increasing from its distal end to its proximal end, and is preferably cone-shaped. Once the balloon is being inflated, the stretched skin and tissue closes over the deflated balloon, then as the balloon is pushed forward, over the insertion means. The gradually increasing diameter of the insertion element enables the skin and tissue to gently stretch so that the punctured opening reaches the distal diameter of the intermediate tubular portion.

In a second embodiment, the insertion means comprises an insertion element comprising two arms for attaching between the distal end of the tubular structure and the proximal end of the deflated balloon, as a spacer and a resiliently deformable portion between the two arms, such that the two arms can gradually move from a first closed position when the balloon is deflated to a second open position when the balloon is inflated. The spacer element is placed at the proximal end of the deflated balloon preferably before insertion of the device into the patient so that it pinches the proximal end of the balloon. The deflated balloon is inserted through the punctured opening. When the skin and tissue reach the spacer element, the balloon is inflated for example by means of an inflating tube. The two arms of the spacer element are gradually pushed outwards as the balloon is inflated until the proximal diameter of the inflated balloon reaches the distal diameter of the intermediary tubular portion. The spacer element can then be removed to allow further insertion of the device, without the skin and tissue becoming trapped.

Preferably, the insertion means comprises an opening for passage of the inflating tube into the interior space of the balloon. In the case of the cone-shaped insertion element, the opening is preferably a centrally located lumen and in the case of the spacer element, the opening can be formed between the two arms of the element.

In a third embodiment, wherein the insertion element comprising a tubular structure coaxial to the balloon, to cover the external surface from the balloon surface to the tubular intermediate structure surface to protect the progression of the device over the tissue. The insertion element has certain flexibility to allow balloon inflation. Once the balloon is sufficiently inflated, and the intermediate tubular structure is advanced over the tissue, the insertion element can be peeled away and consequently removed.

According to a second aspect of the invention, there is provided a medical device, such as a tracheostomy device, comprising a tubular portion and a dilating balloon at the distal end of the tubular portion. The device further comprises a dilating system as described above.

Preferably, the balloon has a reverse truncated cone shape, wherein the distal end diameter is larger than the proximal end diameter, when the balloon is inflated so as to generate a thrust towards the interior of the trachea. More preferably, the balloon has a portion which is active during the dilation phase and which has a reverse truncated cone shape, wherein the distal end base diameter is larger than the proximal end diameter, such that when said balloon is inflated a thrust towards the interior of the trachea is generated.

Preferably, the proximal part of the balloon is partially received inside the tracheal tube. Preferably, the wall of the inflated balloon is tightly connected to the tracheal tube thereby helping the device's forward movement in the trachea and also acting as an inserter tool. Preferably, the proximal profile of the balloon can wrap, for short distance, the outside of the tracheostomy tube, ensuring continuity for the system and providing an extra thrust for the introduction of the device in the trachea;

In a preferred embodiment, the inflating tube ends in the most distal part of the balloon so that inflation of the balloon starts from the tip of the balloon, i.e. from inside the trachea.

Preferably, the distal end of a handle is secured to the proximal end of the tubular portion using a twist-lock connector, a slip-in connector and/or two hooked arms which confer robustness to the whole assembly and allow an easy detachment after insertion of the tracheal tube.

In a preferred embodiment, the handle grip is placed across the device in a "T" shape, i.e. orthogonally relative to the assembly, in order to improved transmission of the effort and control of the device.

The tubular portion may be curve shaped or straight shaped to support and receive a straight or a curved tracheostomy tube, respectively. Preferably, the tracheostomy tube is arranged and constructed so that it firmly grips the intermediate tubular portion and the device after the dilating phase is used also as insertion device.

According to a third aspect of the invention, there is provided a set for tracheostomy comprising the device as described above and a dilating for facilitating the introduction of the device comprising at its outer part two opposite scalpel blades. In a preferred embodiment, the blades are at an angle of 180°.

According to a fourth aspect of the disclosure, there is provided a method for performing a tracheostomy, comprising the steps of (a) forming a punctured opening in the patient's tracheal wall with a needle; (b) inserting a guide wire through the needle bore and withdrawing the needle; (c) optionally, dilating the opening using a dilating; (d) inserting the device as described above and comprising a tracheostomy tube and the balloon in its deflated state along the guide wire; (e) inflating the balloon to obtain the opening of a gap between the tracheal rings, (f) introduction of the tracheostomy tube inside the trachea; and (g) deflating of the balloon, and removing the device.

### DETAILED DESCRIPTION OF THE INVENTION

The invention is further described with reference to the following, non-limiting embodiments shown in the following figures:
FIG. 1 shows a longitudinal view of a device according to the present invention;
FIG. 2 shows a longitudinal sectional view of a dilator for use with a device according to the present invention;
FIG. 3 shows a longitudinal sectional view of a tubular intermediate portion of the device shown in FIG. 1;
FIGS. 4A-4F shows the stages of a tracheostomy procedure using a device according to the present invention;
FIG. 5 shows a longitudinal sectional view of the active part of the dilating balloon of the device of FIG. 1, with a reverse truncated cone shape, inflated and with the force vector Q;
FIG. 6 shows a longitudinal sectional view of a device according to the present invention, with a T-shaped handle, and for use with a straight tracheal tube;
FIG. 7 shows a longitudinal view with the dilating balloon inflated, with the proximal section able to wrap and project beyond the distal edge of the intermediate tubular portion;
FIG. 8A-8B show an example of a first dilatation system according to the present invention comprising a cone shaped insertion element; and
FIG. 9A-9D shows examples of the second dilation system in closed and open position;
FIGS. 10A-10F shows the stages of a tracheostomy procedure using a device according to the present invention, comprising a spacer element;
FIGS. 11A-11D shows examples of the third dilation system according to the present invention comprising a peelable insertion elememt;
FIG. 12A-12F shows the stages of a tracheostomy procedure using a device according to the present invention, comprising a peelable insertion element.

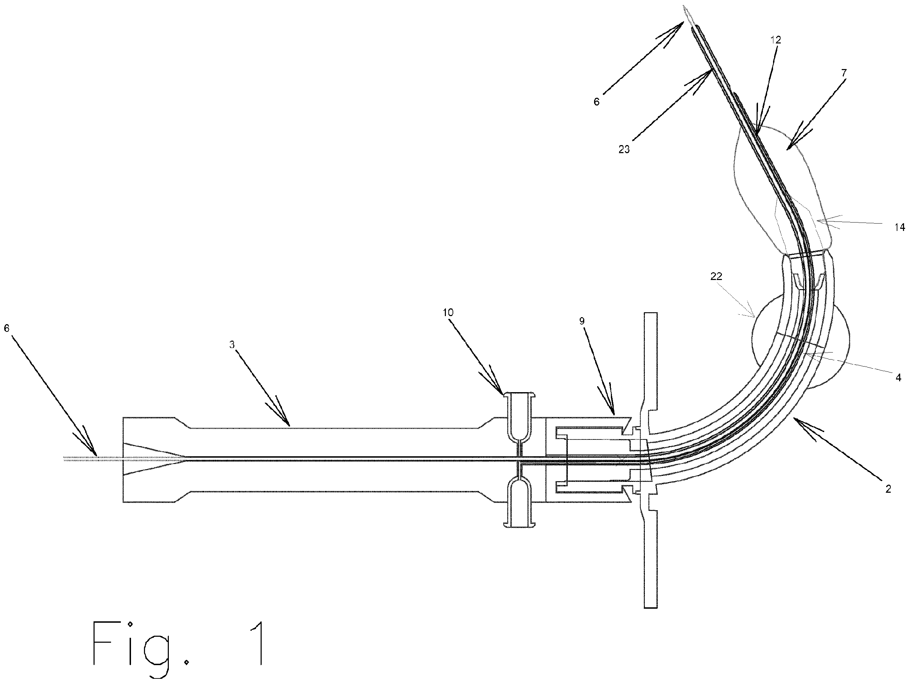

With reference to FIG. 1, an example of the device (1) for percutaneous positioning of a tracheostomy tube (2) according to the present invention comprises, starting from its proximal part, a handle (3), an intermediate tubular portion (4) receiving an inflating tube (5) for inflating a dilating balloon and a guide wire (6), and the dilating balloon (7) in the form of a truncated cone.

Some advantageous characteristics of the device shown in the figures are:
- the balloon (7) plays an active role during the dilating phase owing to its reversed truncated cone shape, i.e. with the distal diameter larger than the proximal diameter next to the tracheostomy tube (2), when the balloon (7) is inflated;
- the proximal end of the balloon (7) contains a cone-shaped tip structure (14), whose function, during the inflation phase, is to prevent skin or paratracheal tissues (29) being caught between the balloon (7) and the distal part of the tracheostomy tube (2), so inhibiting its forward movement inside the trachea.
- the proximal part of the balloon (7) is partly inside the tracheal tube (2) and so is firmly joined to the tracheal tube (2) when inflation is complete.
- d) the proximal part of the dilating balloon (7), in addition to entering the tracheostomy tube (2), may have a special shape enabling it to wrap and extend beyond the tube's distal part, imparting to the (balloon-tube) system greater continuity and preventing the skin (29) becoming trapped between the tube (2)and the balloon (7). The balloon (7) is designed to provide extra thrust for insertion of the tube (2) into the trachea.
- the inflating tube opens in the distal part of the balloon (12), allowing inflation to start inside the trachea.

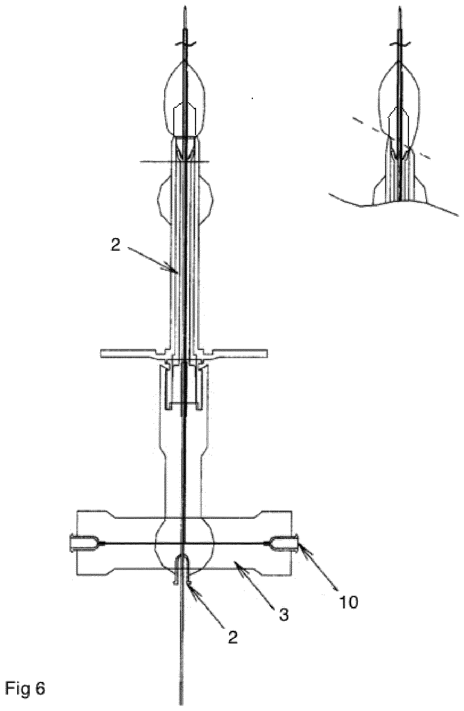

The handle (3) is equipped with a connection to the tracheal tube. The handle (3) of the device (1) in its distal part may be secured to the proximal end of the tracheostomy tube (2) via a twist-lock, slip-in connector and/or is locked by two hooked arms (9). The handle grip can be oriented longitudinally (see FIG.1) or preferably transversely (see FIG. 6) to the device, thereby forming a T-shaped device (1). The handle (3) enables the device (1) to be controlled easily and accurately.

The handle (3) allows the device (1) to be manipulated and may be fitted with Luer adaptors (10) in fluid connection with the balloon's inflating tube. This means that either the left or right hand can be used, allows the connection of a one-way valve or (ON - OFF) tap, and ensures versatility of use and the best possible safety during inflation and deflation.

Additionally, the handle (3) may be fitted with an opening for the guide-wire (6) tube. This opening may have a Luer adaptor to supply the patient with oxygen, if necessary.

The shape of the intermediate tubular portion (4), preferably cylindrical, and the material of which it is made should provide strength, especially during insertion of the tracheostomy tube (2). In a preferred embodiment, the intermediate tubular portion (has a central part, preferably cylindrical in shape and made of a resistant plastic. This arrangement should be large enough to allow easy passage inside the tracheostomy tube. This structure also serves to contain the inflating tube and the guide-wire tube (6), and distally it supports the dilating balloon (7).

The central part within the intermediate tubular portion (4) of the device may be used to:
- contain the inflating tube and the guide-wire tube (6);
- support the tracheostomy tube (2). Hence the dimensions of this structure should allow its easy movement within the tube (2);
- support and hold the dilating balloon (7) in its distal part;

The central part may be curved in its longitudinal plane, if a curved tracheostomy tube is used (see FIG. 1), or straight, if an equipped straight tracheostomy tube (2) is used (see FIG. 6). FIG. 6 shows a device (1) allowing the insertion of an equipped straight tracheostomy tube (2). The equipped tracheostomy tube is flexible and adapts to the curvature after it has been positioned in the trachea. In this case, the device has a straight central section (4). The general aspects of the curved device remain the same, with the only difference being the straight central section.

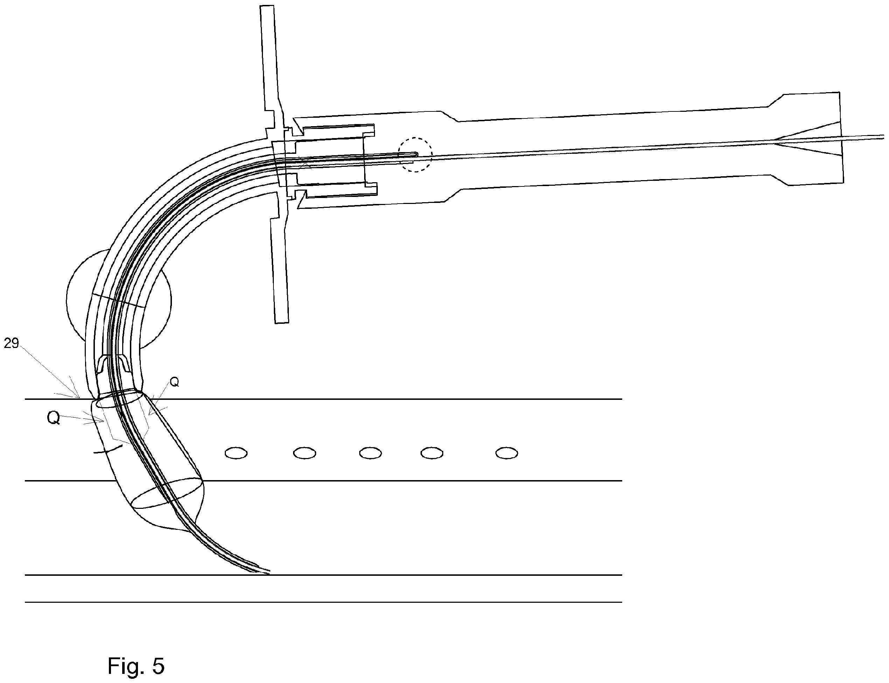

The distal part of the device (1) comprises the dilating balloon (7), partly (e.g. 10 - 20 mm) inside the tracheal tube (2). The dilating balloon (7) branches off from the tube (2) with a diameter which is for example 1 - 3 mm larger than the tube's external dimensions. The dilating balloon (7) starts to inflate at its distal side, with the exit (12) of the inflating tube adjacent to the tip of the balloon (7). The dilating balloon (7) is preferably in the shape of a truncated cone (see for example FIG. 5), with its base at the distal end of the balloon and its top adjacent to the tracheal tube. This, together with inflation, enables a gap to be opened between the tracheal rings. The length of the balloon (7) depends on the patient's gender, age and build; it usually ranges between 3 and 6 cm.

The length of the balloon varies according to the size of the tracheal tube (2). The width of the balloon is preferably conveniently larger than the external diameter of the tracheostomy tube (2), i.e. between 1 and 5 mm.

The dilating balloon (7) is made of a material able to maintain its original shape (i.e. non compliant) at a pressure around 588399-784532 Pa (i.e. 6-8 atmospheres)) without become deformed or breaking. Examples are polymer materials like nylon or PET.

An important innovation in the device (1) is the balloon's shape and, in particular, its active part during the dilating phase. Indeed, it has the shape of a reverse truncated cone, i.e. with its smaller diameter proximal to the tracheal tube (2) and its larger diameter located distally. This is the best possible shape for entry into the trachea, ensuring less resistance and less trauma to the patient's tracheal tissues.

The notion of designing a dilating balloon (7) in the shape of a truncated cone also obeys the formula P = 2 q tg alpha, where alpha is the angle of a right-angled triangle of height 15 - 20 mm and base 2 - 3 mm, at the end of the dilating phase. An angle alpha of 4 - 6 degrees each side is enough to produce a device thrust force of 49033,25-98066,50 Pa (i.e. 49033,25-98066,50 Pa (i.e. 0.5 - 1 atmospheres (or kilo) per square centimetre, when P is equal to 6 atmospheres (FIG. 5, truncated cone with an angle of 5° - 6° degrees, vector Q degrees, inwardly). (P = force opening the tracheal wall, Q = resistance to opening the wall).

The balloon (7) with a truncated conical shape is extremely effective at dilating the tracheal rings, with the diameter of its distal end being larger than that of its proximal end. This shape is more obvious in the active part of the balloon (7).

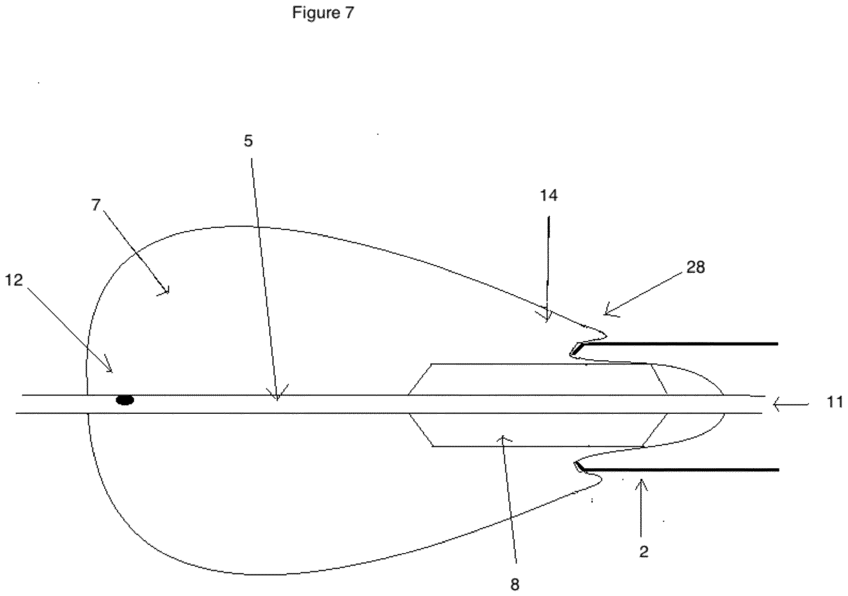

To facilitate further the passage and progression of the tube (2) inside the trachea, the proximal section of the balloon (7) can wrap (28) and project beyond the distal end of the tracheostomy tube, imparting continuity to the balloon - tube system and preventing the skin (29) from being caught between the two structures, in addition to providing a further force for introducing the device (1) into the trachea (FIG. 7).

The proximal part of the dilating balloon (7) enters 1 - 2 centimetres into the tracheostomy tube (2), allowing the inflated balloon (7) to adhere perfectly and firmly to the tracheostomy tube (2) which is thus secured robustly between the handle and the balloon (7), imparting strength and support to the whole device (FIG. 1).

The inflating tube ends (12) at the distal end of the balloon (7) so that, during inflation, the balloon (7) is dilated within the trachea and generates a force assisting the tracheostomy tube (2) to enter the trachea. The dilating balloon applies its dilative force from its distal end, inside the trachea, compressing the tracheostomy tube (2) against the tissues and cartilage rings of the trachea. Since inflation of the balloon starts distally, it will open a gap between the tracheal rings from inside, with the point of least resistance being an arched structure.

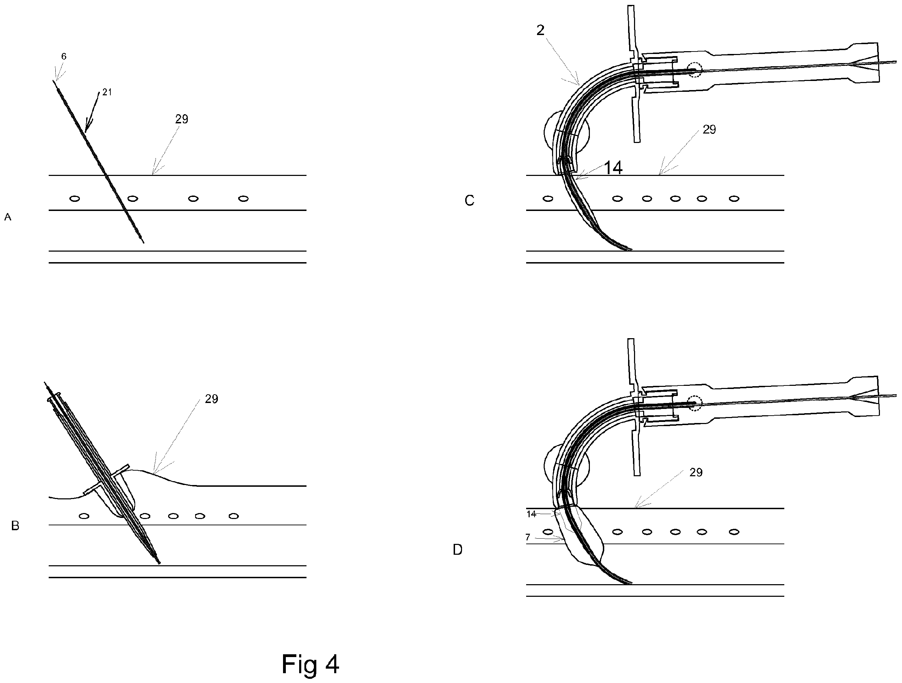

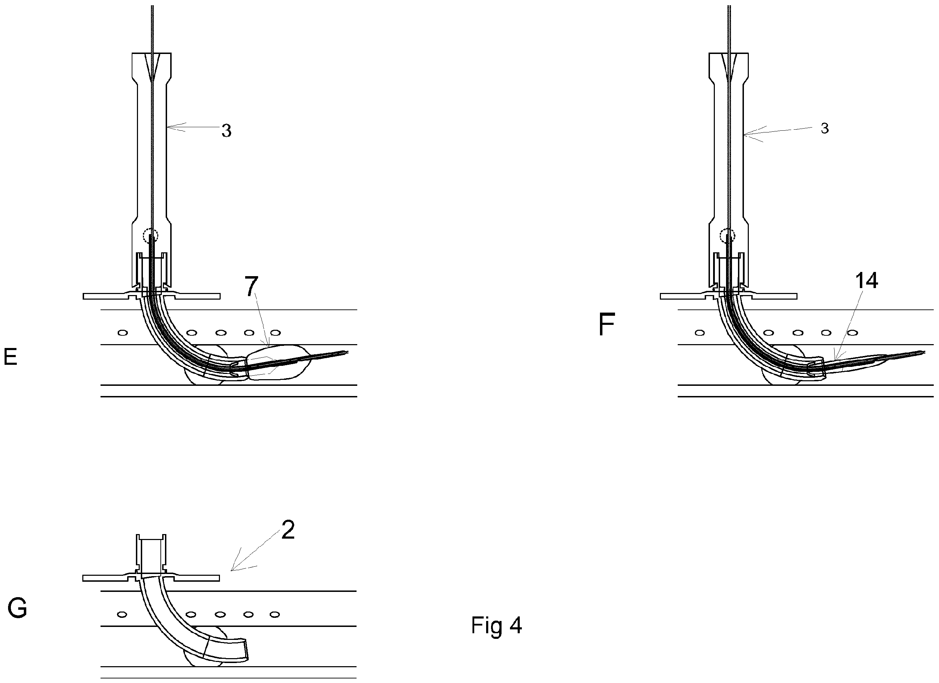

To inflate the balloon (7) with a (sterile physiological) fluid a device such as one comprising a screw syringe is used. To ensure greater safety, the inflating device can be fitted with a manometer and a discharge valve. As inflation of the balloon (7) starts, the device (1) with the tracheal tube (2) is pushed against the tracheal rings, compressing the tracheal tissues between the balloon and the tube, and the inflation pressure makes a gap in the tracheal rings. The insertion element (14) located inside the balloon or, alternatively, the spacer (15) both described below will prevent the skin (29) getting caught between the balloon and the tube, obstructing its progress. The proximal part of the balloon (7) projecting beyond the tube will ease the passage of the tube through the tracheal tissues opened by the balloon (7). Once a gap has been opened in this way between the tracheal tissues, the entire device (1) with the tube (the inflated balloon (7) also acts as an inserter) will be inserted into the trachea. When the tracheal tube (2) has been inserted, the balloon (7) is deflated, the tube is detached from the handle (3) and the device (1) removed, leaving the tracheostomy tube (2) in place (FIG. 4A-4F).

The diameter of the proximal part of the balloon (7) adjacent to the tracheal tube (2) is several millimetres larger than that of the tracheal tube (FIG. 1). The balloon is partially arranged inside the distal end of the tracheal tube (2) so that, when it is inflated, it rigidly fixes in position: the tracheal tube (2) and the handle (3), enabling precision control of the device (FIG. 1).

The tracheostomy tube (2) is firmly anchored to the device (1) and becomes an integral part of the system, thereby enabling it to be inserted into the trachea with the inflated balloon (7) after the dilating phase. Compared with other systems, then, the device (1) is both a dilator and a means of inserting the tracheostomy tube (2) into the trachea. This makes the procedure much quicker, with less time taken for insertion than in other systems. Owing to its truncated cone shape (see e.g. FIG. 5), the inflated balloon (7) keeps the tracheal tissues compact and produces a force which assists insertion of the tracheal tube (2). Thanks to the tapering design of the central section, once the tube (2) has been inserted, the balloon (7) deflated and the handle (3) taken off the connectors, the device (1) can be easily removed, leaving the tracheostomy tube (2) in the trachea (FIG. 4E-4F).

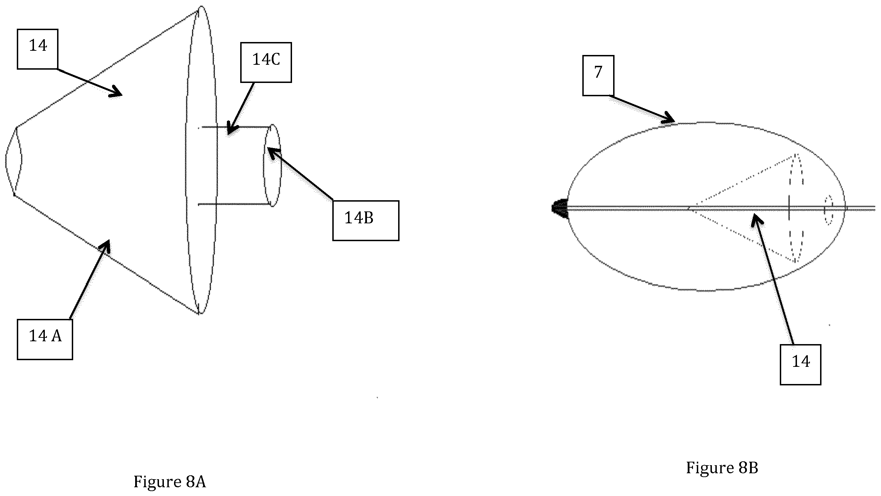

A major innovation and improvement is the location of an insertion element (14) (see FIG. 8A-8B), for example a cone-shaped tip structure (14A) of a solid material (such as plastic) inside and/or adjacent the proximal part of the dilating balloon (7). This cone (14) can have a variable length (for instance 10 - 15 mm), to be adjusted proportionally to the balloon length, allowing the balloon (7) to be inserted partially in the trachea before its inflation, and a variable diameter (for instance 6 mm) according to the tracheotomy tube diameter in order to warranty a smooth transition during the insertion process. The cone-shaped structure (14) is positioned axially relative to the balloon (7), and has at least an internal lumen (14B) to contain the balloon's inflating tube (5) and the guide-wire tube (6) and projects (14C) beyond the distal part of the tracheostomy tube (2). The proximal part of the balloon (7), once inflated, (28) wraps the distal part of the tube (2), thereby helping to easily insert the device (1) into the trachea.

The function of the insertion element (14), during the inflation phase, is to prevent skin or paratracheal tissues (29) being caught between the balloon (7) and the distal part of the tube (2), thereby inhibiting its forward movement inside the trachea. The risk of the skin (29) becoming trapped between the proximal end of the balloon and the distal end of the tube is due to the fact that the deflated balloon (7) has a diameter of 3 - 4 mm whereas the external diameter of the tube (2) can range between 10 and 14 mm. The difference in size between the deflated balloon (7) and the tube (2) means that a 15 - 20 mm incision has to be made in the skin to prevent the skin (29) becoming trapped between the two items. The function of insertion element (14) inside the balloon (7) is to widen the base of the balloon (7) near the distal part of the tube (2), pushing the tissues outwards while the balloon (7) is deflated or during the initial inflation phase. This solid structure (14) allows the balloon inflating tube (5) and the guide-wire tube (6) to pass through it. The diameter of this structure depends on the internal diameter of the tube and must be sufficient to allow easy passage together with the balloon inside the tracheostomy tube (2) (FIG. 1).

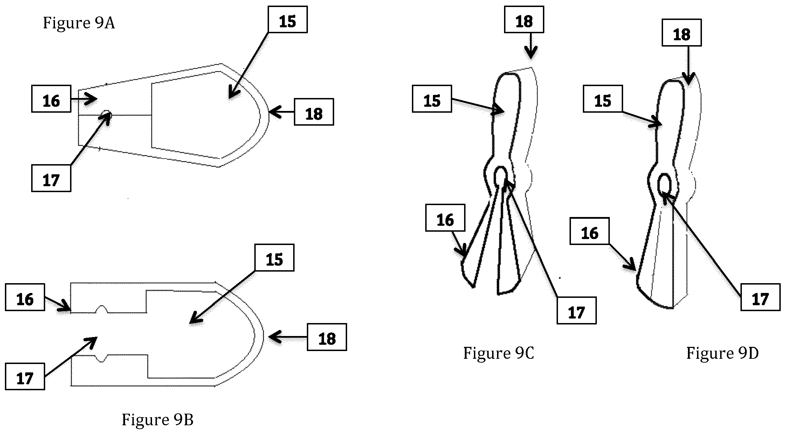

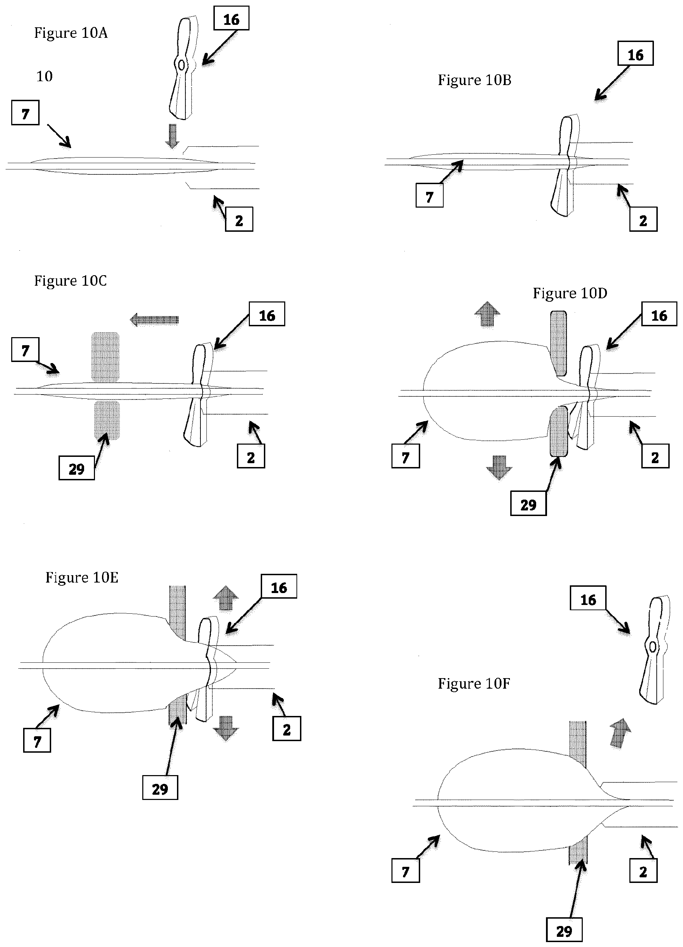

Alternatively or in conjunction with the insertion element (14), a spacer element (15) has been designed (see embodiment on FIG. 9A-9D showing the closed and open positions), which can be divided longitudinally into two parts, prongs or arms 16) with a central hole (17) through which the deflated dilating balloon (7) is inserted as far as the tube. The opening is large enough to allow the deflated balloon (7), but not the tube (2), to pass through. The two parts (16) of the spacer (15) are held together with a spring (18), and when the balloon (7) inflates it has sufficient force to allow it to open. The function of this spacer (15), just a few mm thick, is to allow the balloon (7) to inflate without the skin or paratracheal tissues becoming trapped between the tube (2) and the balloon (7) before the latter is inflated up to the tube (2). This idea is easily understood if clothes pegs are envisaged. The deflated balloon (2) is inserted into the central hole (17) of the spacer (15) which is made to slide until it touches the tracheostomy tube (2). The deflated balloon (7) is then inserted along the guide wire (6) into the trachea. As the balloon (7) inflates, the device with the tube (2) is pushed by the pressure of the balloon (7) until it reaches the spacer (15). Further inflation will expand the balloon (7) until it achieves the external dimensions of the tube (2) before overcoming the resistance in the spring (18) and opening the spacer (15). Designed in this way, the mechanism prevents the patient's skin becoming caught between the balloon (7) and the tube (2), blocking the latter's passage. Each stage described above is shown in FIGS. 10A-10F.

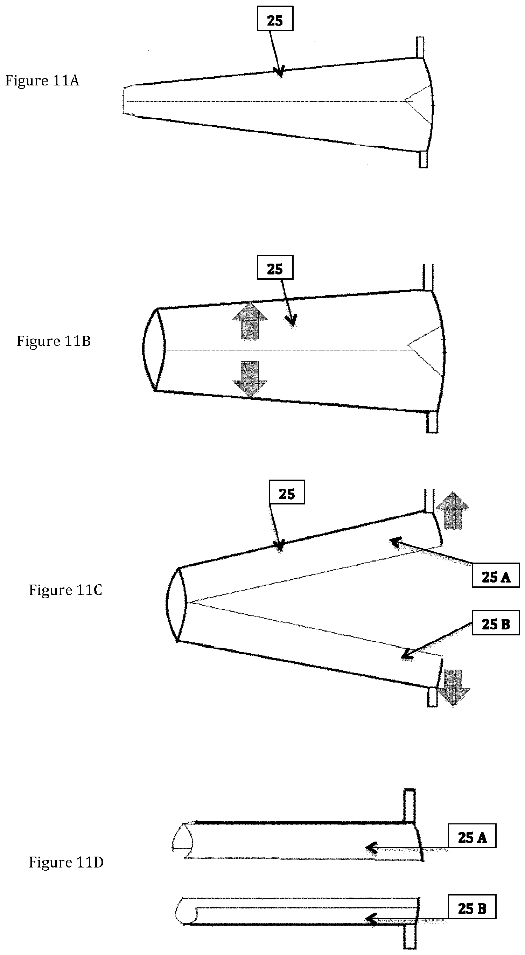

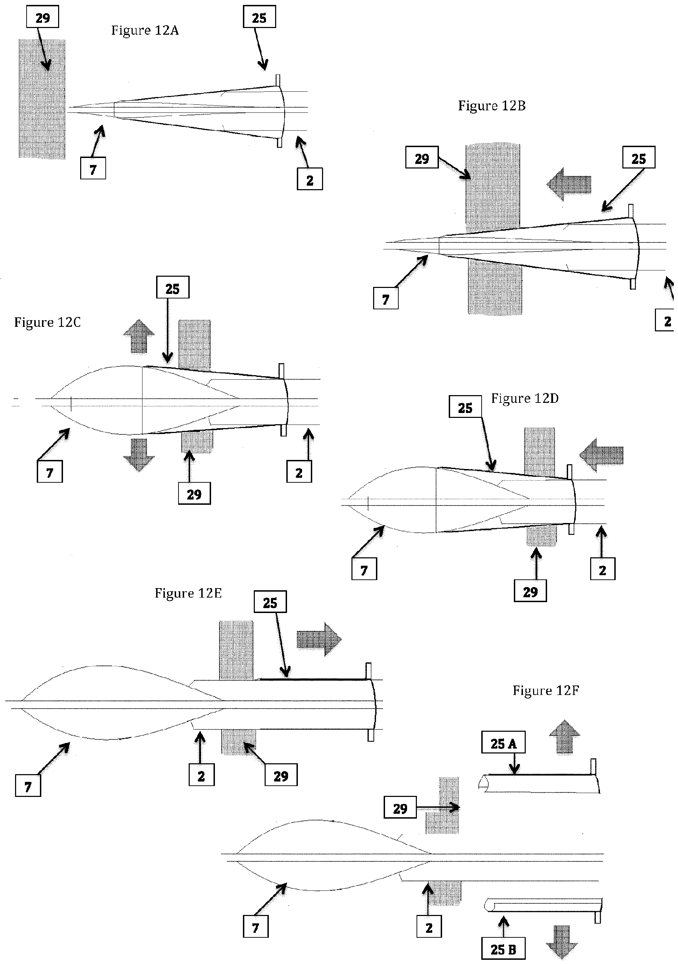

A third dilatation system according to the invention is shown in FIGS 11A-11D and FIGS. 12A-12F comprising a peelable insertion element (25). The peelable insertion element (25) comprises two arms (25A, 25B), which are detachably and can be connected to each other at their distal end. For example, each arm (25A, 25B) of the insertion element (25) is shaped like one half of a longitudinally cut tubular element. The distal end of each arm (25A, 25B) describes a semi-circle. The ends of the semi-circle defined by the distal end of arm 25A are clipped to and/or overlayed onto the ends of the semi-circle defined by the distal end of arm 25B, so that the distal end of arms (25A, 25B) rest around the periphery of the deflated balloon and the proximal end of the arms rest on the distal end of the tube (2) (FIG. 10A). As the balloon (7) is inflated, pressure is exerted onto the distal ends of the arms (25A, 25B) (FIG. 12C), which will eventually detach from each other (FIG.12F) when the balloon (7) is fully inflated, the tubular structure is progressed, and there is no risk of skin or tissues becoming trapped. The detached arms (25A, 25B) can be peeled away and discarded.

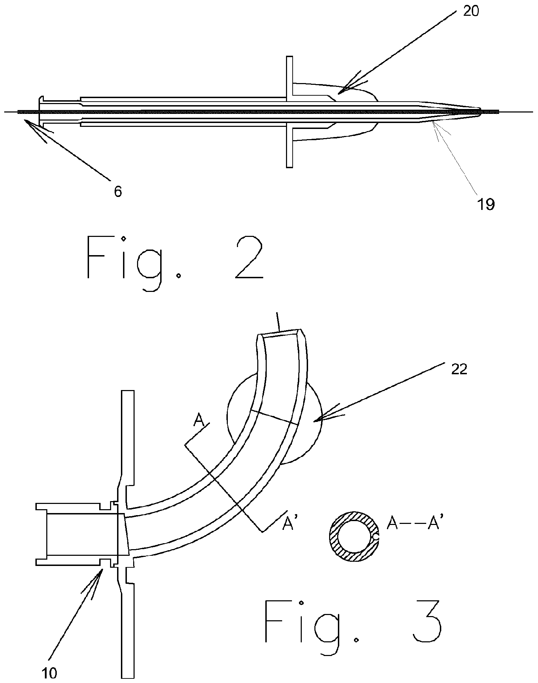

The tracheostomy procedure may involve the use of dilator (19) (FIG. 2) equipped with two scalpel blades (20) arranged opposite each other preferably at an angle of 180°. The dilator (19) is passed along the guide wire (6). It is preferably 4 - 5 mm in diameter and 10 - 11 centimetres long, including the handle of around 6 cm (FIG. 2), with two opposed scalpel blades (20), and it should preferably be made of a hydrophobic plastic. This tool is used to facilitate insertion of the deflated balloon. The purpose of this dilator (19) is to produce a precise incision in the skin as it moves along the guide wire (6), before the deflated dilating balloon (7) is positioned.

### EXAMPLE OF APPLICATION

As illustrated by FIGS 4A to 4F, an example of a tracheostomy procedure using the dilatation system according to the invention involves:
a) positioning of a needle (21)or needle cannula, preferably between the second and third tracheal rings, sufficiently large to allow a guide wire (6) to pass though;
b) insertion of a flexible metal guide wire (6) through the needle. The needle is then removed, leaving the wire (6) in place for its use as a guide in subsequent steps;
c) insertion of the dilator with scalpel blades (20) to create a gap large enough for insertion of the deflated dilating balloon (7);
d) insertion along the guide wire (6)of the surgical device (1) fitted with a tracheostomy tube (2) secured to the handle (3). The deflated and well lubricated dilating balloon (7) of the device (1) is pushed towards the tracheal wall as far as the tip of the cone (14) inside the balloon (7);
f) inflation of the dilating balloon (7) ensures dilatation of the tracheal rings and opening of a gap. The balloon's special shape and its inflation, which starts inside the trachea, thrusts the tracheostomy tube (2) towards the trachea. The presence of the conical device (14) inside the balloon moderates the thrust and helps to spread the tissues, thereby preventing them from becoming trapped between the tube (2) and the balloon (7);
g) attainment of an inflation pressure of 6 - 10 atmospheres and, when dilatation is complete, the tracheostomy tube (2) is pushed into the trachea together with the balloon;
h) once the tracheostomy tube (2) is positioned, the dilating balloon (7)is deflated and the device (1) removed after the handle (3) has been detached from the tube (2).

The procedure can be carried out with the patient intubated or ventilated, with an automatic respirator and with secure access to the airways, just as in emergency situations. This procedure is intended for doctors who are expert in resuscitatory and emergency techniques or are otherwise practised in managing the airways. The patient must lie supine and, if possible, preferably with the neck hyper-extended. The working area must have been prepared with sterile cloths and the skin disinfected; vital signs should be monitored. The procedure can be assisted by means of an endoscope through the orotracheal tube, if present. Insertion of the device fitted with the tracheal tube is preceded by an injection of local anaesthetic between the tracheal rings, usually the second and the third. This is done with a cannula needle connected to a 10 ml Luer syringe; inhaled air will confirm correct access to the trachea. When the needle has been inserted, a guide wire with a J tip is introduced and the needle then removed (FIG. 4A); the wire will be used as a guide in the subsequent steps (FIG. 4B-4F).

### Reference numerals

- 1: Tracheostomy device
- 2: Tracheostomy tube
- 3: Handle
- 4: Intermediate tubular portion
- 5: Inflating tube
- 6: Guide wire
- 7: Dilating balloon
- 8: Cone shaped insertion element
- 9: Hooked arms to connect the handle
- 10: Luer connectors to inflating tube
- 11: Opening for guide wire tube
- 12: Exit of the inflating tube
- 14: Cone/Insertion element
- 14A: Cone shaped tip
- 14B: Lumen
- 14C: Extension
- 15: Spacer/Insertion element
- 16: Arms of spacer element
- 17: Opening through spacer element
- 18: Spring
- 19: Dilator
- 20: Scalpel blades
- 22: Second balloon
- 23: Guide wire tube
- 25: Peelable/Insertion element
- 25A/B: Arms of peelable insertion element
- 28: proximal part of the balloon wraps the distal part of the tube
- 29: skin or paratracheal tissues

## Claims

1. A dilatation system for a medical device comprising a tubular portion (4) and a dilating balloon (7) at the distal end of the tubular portion (4), said dilatation system comprising an insertion element (14) for preventing skin or tissue to be trapped between the distal end of the tubular portion (4) and the proximal end of the deflated balloon (7), **characterised in that** the insertion element (14) is located inside the dilating balloon (7) and has a diameter increasing from its distal end to its proximal end.

2. The dilatation system of claim 1, wherein the insertion element (14) is located at the distal end of the tubular portion (4).

3. The dilatation system of claim 1 or 2, wherein the insertion element (14) is located co-axially relative to the tubular portion (4).

4. The dilatation system of claim 1, wherein the insertion element (14) is cone-shaped.

5. The dilatation system of any one of claim 1 to 3, wherein the insertion element (14) comprises a spacer element (15) comprising two arms (16) for attaching to the proximal end of the deflated balloon (7) and a resiliently deformable portion between the two arms (16), such that the two arms can gradually move from a first closed position when the balloon (7) is deflated to a second open position when the balloon (7) is inflated.

6. The dilatation system of any one of the preceding claims, wherein the insertion element (14) comprises an opening for passage of said inflating tube(5) into the balloon (7).

7. A medical device comprising
a tubular portion (4); and
a dilating balloon (7) at the distal end of the tubular portion (4);
said device further comprising a dilating system according to any one of claims 1 to 6.

8. The device according to claim 7, wherein the balloon (7) comprises a portion active during the dilation phase having a reverse truncated cone shape, wherein the distal end base diameter is larger than the proximal end diameter.

9. The device according to claim 7, wherein the proximal part of the balloon (7) is partially received inside the tubular portion (4).

10. The device according to claim 7, wherein the wall of the inflated balloon (7) is tightly connected to the tubular portion (4).

11. The device according to claim 7, wherein the proximal profile of the balloon (7) is constructed to that it partially wraps the distal end of the tubular portion (4) or a tracheotomy tube (2).

12. A device according to claim 9, further comprising an inflating tube (5) for inflating the balloon (7); wherein the inflating tube (5) ends in the distal part of the balloon (7) so that inflation of the balloon (7) starts from the distal end of the balloon (7).

13. The medical device according to any one of claims 7 to 12, wherein the device is a tracheostomy device.

14. A dilatation kit comprising the device according to any one of claims 7 to 12 and further comprising a dilator (19) for facilitating the introduction of the device comprising at its outer part two opposite scalpel blades.

## Patentansprüche

1. Dilatationssystem für eine medizinische Vorrichtung, umfassend einen röhrenförmigen Abschnitt (4) und einen Dilatationsballon (7) am distalen Ende des röhrenförmigen Abschnitts (4), wobei das Dilatationssystem ein Einführelement (14) zum Verhindern, dass Haut oder Gewebe zwischen dem distalen Ende des röhrenförmigen Abschnitts (4) und dem proximalen Ende des entleerten Ballons (7) eingeklemmt wird, umfasst, **dadurch gekennzeichnet, dass** sich das Einführelement (14) in dem Dilatationsballon (7) befindet und einen Durchmesser aufweist, der von seinem distalen Ende zu seinem proximalen Ende zunimmt.

2. Dilatationssystem nach Anspruch 1, wobei sich das Einführelement (14) am distalen Ende des röhrenförmigen Abschnitts (4) befindet.

3. Dilatationssystem nach Anspruch 1 oder 2, wobei sich das Einführelement (14) koaxial relativ zu dem röhrenförmigen Abschnitt (4) befindet.

4. Dilatationssystem nach Anspruch 1, wobei das Einführelement (14) kegelförmig ist.

5. Dilatationssystem nach einem der Ansprüche 1 bis 3, wobei das Einführelement (14) ein Distanzelement (15) umfasst, das zwei Arme (16) zum Anbringen an dem proximalen Ende des entleerten Ballons (7) und einen elastisch verformbaren Abschnitt zwischen den zwei Armen (16) umfasst, sodass sich die zwei Arme allmählich von einer ersten, geschlossenen Stellung, wenn der Ballon (7) entleert ist, zu einer zweiten, offenen Stellung, wenn der Ballon (7) aufgeblasen ist, bewegen können.

6. Dilatationssystem nach einem der vorangehenden Ansprüche, wobei das Einführelement (14) eine Öffnung zum Hindurchgelangen der Aufblasröhre (5) in den Ballon (7) umfasst.

7. Medizinische Vorrichtung, die Folgendes umfasst:
einen röhrenförmigen Abschnitt (4); und
einen Dilatationsballon (7) am distalen Ende des röhrenförmigen Abschnitts (4);
wobei die Vorrichtung weiter ein Dilatationssystem nach einem der Ansprüche 1 bis 6 umfasst.

8. Vorrichtung nach Anspruch 7, wobei der Ballon (7) einen während der Dilatationsphase aktiven Abschnitt mit einer umgekehrten Kegelstumpfform umfasst, wobei der Basisdurchmesser am distalen Ende größer ist als der Durchmesser am proximalen Ende.

9. Vorrichtung nach Anspruch 7, wobei der proximale Teil des Ballons (7) teilweise in dem röhrenförmigen Abschnitt (4) aufgenommen ist.

10. Vorrichtung nach Anspruch 7, wobei die Wand des aufgeblasenen Ballons (7) fest mit dem röhrenförmigen Abschnitt (4) verbunden ist.

11. Vorrichtung nach Anspruch 7, wobei das proximale Profil des Ballons (7) derart aufgebaut ist, dass es das distale Ende des röhrenförmigen Abschnitts (4) oder einer Tracheotomieröhre (2) teilweise umhüllt.

12. Vorrichtung nach Anspruch 9, weiter umfassend eine Aufblasröhre (5) zum Aufblasen des Ballons (7); wobei die Aufblasröhre (5) im distalen Teil des Ballons (7) endet, sodass das Aufblasen des Ballons (7) vom distalen Ende des Ballons (7) aus beginnt.

13. Medizinische Vorrichtung nach einem der Ansprüche 7 bis 12, wobei es sich bei der Vorrichtung um eine Tracheostomievorrichtung handelt.

14. Dilatationsset, umfassend die Vorrichtung nach einem der Ansprüche 7 bis 12 und weiter umfassend einen Dilatator (19) zum Erleichtern des Einführens der Vorrichtung, der an seinem äußeren Teil zwei einander gegenüberliegende Skalpellklingen umfasst.

## Revendications

1. Système de dilatation pour un dispositif médical comportant une partie tubulaire (4) et un ballonnet de dilatation (7) au niveau de l'extrémité distale de la partie tubulaire (4), ledit système de dilatation comportant un élément d'insertion (14) servant à empêcher tout emprisonnement de la peau ou du tissu entre l'extrémité distale de la partie tubulaire (4) et l'extrémité proximale du ballonnet dégonflé (7), **caractérisé en ce que** l'élément d'insertion (14) est situé à l'intérieur du ballonnet de dilatation (7) et a un diamètre qui va en augmentant depuis son extrémité distale jusqu'à son extrémité proximale.

2. Système de dilatation selon la revendication 1, dans lequel l'élément d'insertion (14) est situé au niveau de l'extrémité distale de la partie tubulaire (4).

3. Système de dilatation selon la revendication 1 ou la revendication 2, dans lequel l'élément d'insertion (14) est situé de manière coaxiale par rapport à la partie tubulaire (4).

4. Système de dilatation selon la revendication 1, dans lequel l'élément d'insertion (14) est en forme de cône.

5. Système de dilatation selon l'une quelconque des revendications 1 à 3, dans lequel l'élément d'insertion (14) comporte un élément d'écartement (15) comportant deux bras (16) servant à des fins de fixation sur l'extrémité proximale du ballonnet dégonflé (7) et une partie déformable de manière élastique entre les deux bras (16), de telle sorte que les deux bras peuvent se déplacer progressivement d'une première position fermée quand le ballonnet (7) est dégonflé à une deuxième position ouverte quand le ballonnet (7) est gonflé.

6. Système de dilatation selon l'une quelconque des revendications précédentes, dans lequel l'élément d'insertion (14) comporte une ouverture à des fins de passage dudit tube de gonflement (5) dans le ballonnet (7).

7. Dispositif médical comportant
une partie tubulaire (4) ; et
un ballonnet de dilatation (7) au niveau de l'extrémité distale de la partie tubulaire (4) ;
ledit dispositif comportant par ailleurs un système de dilatation selon l'une quelconque des revendications 1 à 6.

8. Dispositif selon la revendication 7, dans lequel le ballonnet (7) comporte une partie active au cours de la phase de dilatation ayant une forme conique tronquée inverse, dans lequel le diamètre de base de l'extrémité distale est supérieur au diamètre de l'extrémité proximale.

9. Dispositif selon la revendication 7, dans lequel la partie proximale du ballonnet (7) est reçue partiellement à l'intérieur de la partie tubulaire (4).

10. Dispositif selon la revendication 7, dans lequel la paroi du ballonnet gonflé (7) est étroitement connectée à la partie tubulaire (4).

11. Dispositif selon la revendication 7, dans lequel le profil proximal du ballonnet (7) est construit de telle sorte qu'il enveloppe partiellement l'extrémité distale de la partie tubulaire (4) ou un tube de trachéotomie (2).

12. Dispositif selon la revendication 9, comportant par ailleurs un tube de gonflement (5) servant à des fins de gonflage du ballonnet (7) ; dans lequel le tube de gonflement (5) se termine dans la partie distale du ballonnet (7) de telle sorte que le gonflage du ballonnet (7) commence depuis l'extrémité distale du ballonnet (7).

13. Dispositif médical selon l'une quelconque des revendications 7 à 12, dans lequel le dispositif est un dispositif de trachéostomie.

14. Trousse de dilatation comportant le dispositif selon l'une quelconque des revendications 7 à 12 et comportant par ailleurs un dilatateur (19) ayant pour objet de faciliter l'introduction du dispositif comportant au niveau de sa partie extérieure deux lames de scalpel opposées.
